# EUROPEAN PATENT SPECIFICATION

(11) **EP 3 246 329 B1**
(45) Date of publication and mention of the grant of the patent: **09.05.2018**
(21) Application number: 16001118.5
(22) Date of filing: 17.05.2016
(51) Int. Cl.: C07D 495/04, C08G 61/12, H01B 1/12

(54) **METHOD FOR THE PREPARATION OF A MONOMER BASED ON THIOPHENE OF FORMULA (2,3-DIHYDROTHIENO [3,4-B][1,4]DIOXIN-2-YL)METHANOL AND THE MONOMER PREPARED IN THIS MANNER**
VERFAHREN ZUR HERSTELLUNG EINES MONOMERS AUF BASIS VON THIOPHEN DER FORMEL (2,3-DIHYDROTHIEN [3,4-B][1,4]DIOXIN-2-YL)METHANOL UND NACH DIESEM VERFAHREN HERGESTELLTES MONOMER
PROCÉDÉ POUR LA PRÉPARATION D'UN MONOMÈRE À BASE DE THIOPHÈNE DE FORMULE (2,3-DIHYDROTHIENO [3,4-B] [1,4] DIOXIN-2-YL) MÉTHANOL ET MONOMÈRE PRÉPARÉ DE CETTE MANIÈRE

(43) Date of publication of application: 22.11.2017
(73) Proprietor: Centrum organické chemie s.r.o., 533 54 Rybitví (CZ)
(72) Inventor: KUBÁC, Lubomír, 533 54 Rybitví (CZ); BESVARÍKOVÁ, Radana, 500 03 Hradec Králové (CZ); HORÁKOVÁ, Petra, 533 07 Pardubice (CZ)
(74) Representative: Sedlák, Jirí

(56) References cited:
- EP-B1- 1 928 000
- WO-A2-2006/073968
- CZ-B6- 301 500
- US-A- 5 111 327
- STEPHAN O ET AL: "ELECTROCHEMICAL BEHAVIOUR OF 3,4-ETHYLENEDIOXYTHIOPHENE FUNCTIONALIZED BY A SULPHONATE GROUP. APPLICATION TO THE PREPARATION OF POLY(3,4-ETHYLENEDIOXYTHIOPHENE) HAVING PERMANENT CATION-EXCHANGE PROPERTIES", JOURNAL OF THE ELECTROANALYTICAL CHEMISTRY, LSEVIER SCIENCE B.V, NL, vol. 443, no. 2, 1 January 1998 (1998-01-01), pages 217-226, XP000995724, ISSN: 0368-1874, DOI: 10.1016/S0022-0728(97)00548-2
- SCHOTTLAND P ET AL: "SYNTHESIS AND POLYMERIZATION OF NEW MONOMERS DERIVED FROM 3,4-ETHYLENEDIOXYTHIOPHENE", JOURNAL DE CHIMIE PHYSIQUE, SOCIETE DE CHIMIE PHYSIQUE, PARIS, FR, vol. 95, no. 6, 1 January 1998 (1998-01-01), pages 1258-1261, XP001013494, ISSN: 0021-7689, DOI: 10.1051/JCP:1998260
- "The Synthesis of EDOT Monomer, and Its Physical and Chemical Properties" In: "PEDOT Principles and Applications of an Intrinsically Conductive Polymer", 1 January 2011 (2011-01-01), CRC Press, Boca Raton, XP055049955, ISBN: 978-1-42-006911-2 pages 47-66, DOI: 10.1201/b10318-6, * citation 20 on page 64 and 27 on page 65; figures 5.1, 5.16 *

## Description

### Field of the invention

The present invention relates to the method for the preparation of a monomer based on thiophene of formula (2,3-dihydrothieno [3,4-b][1,4]dioxin-2-yl)methanol and this monomer.

### Background of the invention

Flexible electronic devices are a dynamic part of industry increasingly connected with the daily life of people. Companies are developing lighting systems based on OLEDs, flexible photovoltaic systems, flexible electrochromic devices, safety cards, labels for goods, smart textiles equipped with sensory systems, smart packaging endowed with memories, and many types of toys. In all these applications conductive polymers as a basic functional layer may play a prominent role. Conductive polymers are commonly used in three ways. One common application is smoothing layers for conductive metal oxides such as tin-doped indium oxide (ITO). Such layers enable better transport of charge by smoothing the rough ITO surfaces thus facilitating the injection of charge into the working layer under concern.

A second application of conductive polymers is conductive layers on flexible materials to substitute conductive metal oxides made form critical and scarce metals. This application requires high conductivity and high transparency. The use of conductive polymers here paves the way for using cost-effective and energy-efficient printing or other wet-chemical coating methods instead of vacuum evaporation technology usually characterized by high energy consumption and introduction (into the substrate to be coated).

The third application of conductive polymers concerns functional layers in electronic systems such as sensors or electrochromic devices. The latter application is based on the ability of conducting polymer layers to change their color upon application of a small voltage. Commonly, electrodes made from thin layers of doped metal oxides (Transparent Conducting Oxides, TCO) are used as current collectors in such devices.

The most common conductive polymer materials applied in electronics are aqueous or alcoholic dispersions of doped polyethylene dioxythiophene / polystyrene sulphonate (PEDOT/PSS). PSS is employed as dopant and polyelectrolyte in these polymers to enable processing in water and other polar solvents and may amount up to 70 %wt. of the total polymer solids. Such dispersions are produced in different physical form, which results in different conductivity, substrate adhesion, layer cohesion or processing characteristics, e.g. for printing, layering, and immersion.

For some applications a method called in-situ chemical oxidative polymerization is used to produce layers directly on the substrate. As pure polythiophene layers are formed by in-situ polymerization (not containing polyelectrolyte, but anions to charge balance the doped material), this method results in layers with better morphology and higher conductivity. However, in-situ polymerized layers have to be rinsed from residual monomers, oligomers, and organic as well as inorganic salts. Upon mixing the monomer with oxidative agents, polymerization starts. Consequently, the mixture shows low stability and pot life. The structure and synthesis of monomers suitable for working electrodes in plastic electrochromic devices is described in EP 1 928 000 B1 and WO 2008/064878.

Thin organic layers on flexible materials, e.g. PET films, distinguished by high conductivity are a basic part of electronic devices such as OLEDs, organic photovoltaic systems, sensoric systems, electrochromic systems etc. In order become industrially viable technology, the conductive layers need to be applied by continuous wet-chemical processing (roll-to-roll coating) or printing technology. Because of their high thermal and chemical stability and as well as high electronic conductivity, conductive polymers, mainly polymers based on polyethylene dioxythiophene (PEDOT) derivatives are first choice.

PEDOT is mostly applied in the form of a doped complex with polystyrene sulphonate (PEDOT/PSS), dispersed in water or alcohols. It is known to prepare thin layers of doped PEDOT and derivatives thereof directly and continuously on flexible substrates, which presents a highly cost-effective and energy- efficient method to prepare functional layers for flexible electrochromic devices. The same process can be useful and advantageously applied for conductive layers in other electronic devices.

Earlier work on polymer films described in WO 2008/064878 shows that the electrochromic performance strongly depends on the purity of the corresponding monomer that has to be free of impurities affecting the film quality. Such impurities can be aliphatic or aromatic hydrocarbons carried over from starting materials, condensation products of starting materials or intermediate products, or isomeric forms of the monomer showing different polymerization rate.

Special types of monomers based on the basic EDOT skeleton are monomers substituted on their side chains as indicated by the formula XII. This monomer is prepared by etherification of the basic monomer, 2,3-dihydrothieno[3,4-b][1,4]dioxin-2-yl)methanol. However, a standard procedure in the course of the preparation results in a mixture of derivatives containing 2,3-dihydrothieno[3,4-b][1,4]dioxin-2-yl)methanol (EDOT MeOH) and 3,4-dihydro-2H-thieno[3,4-b][1,4]dioxepin-3-ol (ProDOT OH), in the ratio 80:20. EP 1928000 describes usage of these compounds in this ration for the preparation of new types of monomers by etherification. The prepared monomers are subsequently used in situ polymerization and for the preparation of an electrochromic system. To prepare the pure ether in agreement with the formula XII the product must be treated with column chromatography with major losses or it is possible to use a different solution of acids, 2-(hydroxymethyl)-2,3-dihydrothieno[3,4-b]-1,4-dioxine-5,7-dicarboxylic acid and 3-hydroxy-3,4-dihydro-2H-thieno[3,4-b][1,4]dioxepine-6,8-dicarboxylic acid, to treat intermediate products before the last reaction stage, i.e. decarboxylation. The reaction yield thus decreases by 20 to 30 % which means significant material loss in the synthesis which uses expensive intermediates and the reaction is thus not industrially viable. WO 2006073968 describes an alternative process for the synthesis of EDOT MeOH. There is a significant risk of polymerization during the synthesis due to the fact that the thiophene cycle in position 2 and 5 is not protected by ethyl-carboxyl units. The process is not sufficiently robust for an industrial realization. An alternative synthesis of pure EDOT MeOH is also disclosed in Journal of the electroanalytical chemistry, Stephan O. et al, 1998, 443, 217-226. Tests of preparation of a conductive layer based on the initial monomer with the formula XII and its in situ polymerization have shown that presence of a monomer based on 3,4-(2'-hydroxy)propylene dioxythiophene with the formula XIII significantly reduces quality of the film, decreases the rate and the contrast of electrochromic transition and it has an overall negative effect on quality of the conductive film. 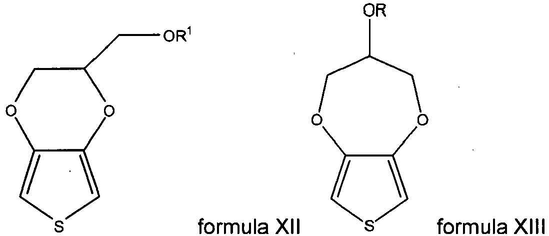 where R¹ is hydrogen or C₁ - C₁₁ linear and/or branched alkylene or alkyl chain.

The purpose of the invention is to create a method for the preparation of a monomer based on thiophene of formula (2,3-dihydrothieno [3,4-b][1,4]dioxin-2-yl)methanol, which is able to eliminate the disadvantages indicated above and to ensure that the monomer prepared in this manner contains only small quantities of impurities and byproducts produced in the reaction, which would make the entire process more economically viable with regard to the expensive raw materials and intermediates.

### Summary of the invention

The disadvantages mentioned above are removed by a method for the preparation of a monomer based on thiophene of formula (2,3-dihydrothieno[3,4-b][1,4]dioxin-2-yl)methanol which produces a mixture of compounds, where the monomer is synthetized in agreement with the following reaction scheme 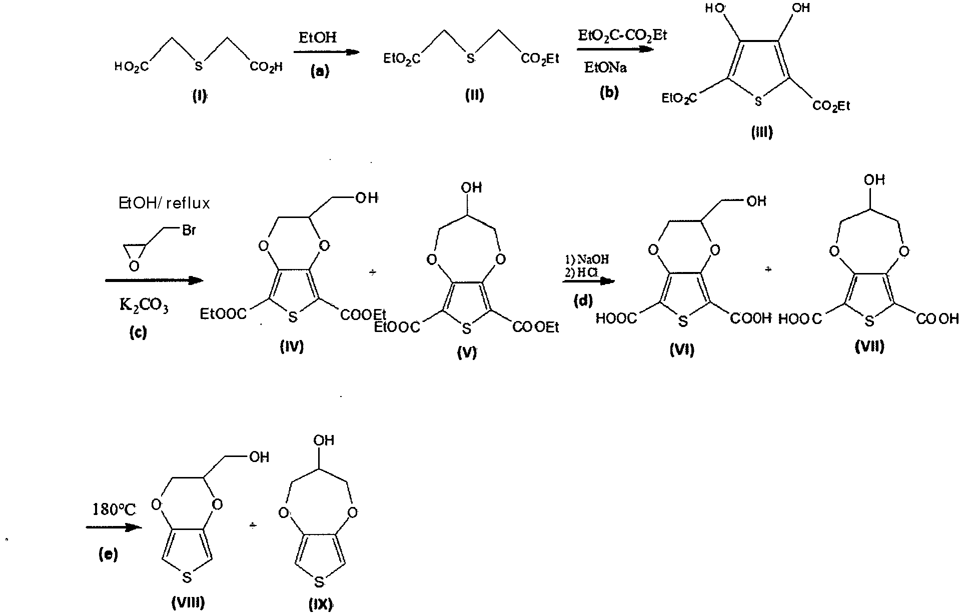 In the step (c) of the reaction a mixture of 3,4-dihydroxy-thiophene-2,5-dicarboxylic acid diethyl ester (III), epibromhydrin and potassium carbonate is used to prepare a mixture of intermediates: diethyl 2,3-dihydro-2-(hydroxymethyl)thieno[3,4-b][1,4]dioxine-5,7-dicarboxylate (IV) and 3-hydroxy-3,4-dihydro-2H-thieno[3,4-b][1,4]dioxepine-6,8-dicarboxylic acid diethyl ester (V) according to the presented invention.

The principle of the present invention consists in the fact that the step (c) of the reaction epibromhydrin and potassium carbonate are dosed in intervals of 120 to 240 minutes into the solution of 3,4-dihydroxy-thiophene-2,5-dicarboxylic acid diethyl ester (III), while the molar ratio of 3,4-dihydroxy-thiophene-2,5-dicarboxylic acid diethyl ester (III) and epibromhydrin is in the range 1:20 to 1:25, and after the dosing is completed the mixture is stirred for the maximum period of 180 minutes, where the mixture of these compounds contains less than0.1 % wt. of impurities of general formula (X, XI) 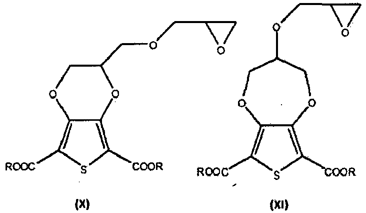 which are produced as products of condensation of a mixture of the compounds (IV and V) with epibromhydrin. The mixture of the compounds (IV and V) is in the steps (d) and (e) used to prepare the resulting monomer (VIII) and also the byproduct 3,4-dihydro-2H-thieno[3,4-b][1,4]dioxepin-3-ol (IX), while the resulting monomer (VIII) contains less than 1 % wt. of the mentioned byproduct (IX).

In order to reduce the content of impurities (X, XI) it is also expedient to wash the mixture of the compounds (IV and V) with one or mixture of the non-polar solvent from the group cyclohexane, toluene, benzene or a mixture thereof.

Advantages of the method of preparation of the monomer based on thiophene of formula (2,3-dihydrothieno [3,4-b][1,4]dioxin-2-yl)methanol hereunder consist particularly in preparation of the monomer (2,3-dihydrothieno [3,4-b][1,4]dioxin-2-yl)methanol with a low byproduct content and with a low content of impurities. Therefore the whole process of preparation of the monomer based on thiophene of formula (2,3-dihydrothieno [3,4-b][1,4]dioxin-2-yl)methanol becomes more economically viable as the input materials or intermediates needed for its synthesis are expensive and hard to get.

### Detailed Description of the Preferred Embodiments

It is assumed that the specific examples of the invention, as described and shown below, are only provided for illustration and they do not mean that the invention is limited to such examples. Experts familiar with the status of the technology are able or will be able, with the use of routine experiments, to find one or more equivalents to the specific examples of execution of the invention as described herein. Even such equivalents shall be included in the scope of the following patent claims.

### Comparative example

### Synthesis of a mixture of (2,3-dihydro-thieno[3,4-b][1,4]dioxin-2-yl)-methanol VIII and 3,4-dihydro-2H-thieno[3,4-b][1,4]dioxepin-3-ol XI

### a) Synthesis of thiodiacetic acid diethyl ester II

Sulphuric acid - 110 ml was slowly added to a mixture of 2 750 ml ethanol and 550 g thiodiglycolic acid (3,66 mol). The mixture was heated at reflux for 10 hours. Thereafter, ethanol was removed under reduced pressure and 200 ml of toluene and 500 ml of water were added. The obtained mixture was put into a separation funnel and the water layer was removed. Next, the organic layer was washed with 700 ml of concentrated sodium carbonate solution and twice by water. Then toluene was removed under reduced pressure from this solution to obtain 580 g of thiodiacetic acid diethyl ester **II** with a yield of 77 %.

### b) Synthesis of 3,4-dihydroxy-thiophene-2,5-dicarboxylic acid diethyl ester III

A solution of sodium ethanolate was prepared by mixing of 3 200 ml absolute (water free) ethanol and 146 g of sodium (6,35 mol) under reflux. A mixture of 285 g thiodiacetic acid diethyl ester **II** (1,38 mol) and 500 g of diethyloxalate (3,42 mol) was then slowly added to a hot solution of sodium ethanolate. Yellow precipitation started. The mixture was left for 1 hour under reflux and was then cooled at 15 °C. The yellow product (diethyl 3,4-dihydroxythiophene-2,5-dicarboxylate disodium salt) was filtered off. The wet cake was transferred into 4 000 ml of water and, under mixing, was heated to 50 °C. 310 ml of hydrochloric acid (3,51 mol) was added and the yellow suspension changed its colour into white. The mixture was left at 50 °C for 30 minutes and then was cooled to 15 °C. The precipitate was filtered off, washed by water and dried at 50 °C. 267 g of diethyl 3,4-dihydroxy-thiophene-2,5-dicarboxylic acid diethyl ester **III** were obtained corresponding to a yield of 76 %.

### c) Synthesis of a mixture of diethyl 2,3-dihydro-2-(hydroxymethyl)thieno[3,4-b][1,4]dioxine-5,7-dicarboxylate IV and 3-hydroxy-3,4-dihydro-2H-thieno[3,4-b][1,4]dioxepine-6,8-dicarboxylic acid diethyl ester V

A solution of 200 g of 3,4-dihydroxy-thiophene-2,5-dicarboxylic acid diethyl ester **III** (0,768 mol) in 2 550 ml of ethanol was prepared by mixing and heating at reflux. 32,8 g potassium carbonate (0,154 mol) in 1 000 ml water was slowly added during 90 minutes together with 375 g of epibromohydrin (2,74 mol). The mixture was left under reflux for 24 hours, then cooled at 30 °C and finally poured into a mixture of 3 500 ml water and 600 ml of hydrochloric acid. The suspension was cooled at 0 °C and mixed for the next 3 hours at this temperature. The precipitate was filtered off, washed by water and dried at 65 °C. 212 g of a mixture of diethyl 2,3-dihydro-2-(hydroxymethyl)thieno[3,4-b][1,4]dioxine-5,7-dicarboxylate **IV** and diethyl 3,4-(2'-hydroxy)propylene dioxythiophene-5,7-dicarboxylate was obtained, corresponding to a yield of 87 %. HPLC analysis proved that the product contained 67 % of diethyl 2,3-dihydro-2-(hydroxymethyl)thieno[3,4-b][1,4]dioxine-5,7-dicarboxylate **IV**, 23 % of 3-hydroxy-3,4-dihydro-2H-thieno[3,4-b][1,4]dioxepine-6,8-dicarboxylic acid diethyl ester **V** and 10 % of unknown impurities. GC/MS identified these impurities mainly to have a molecular mass of M = 372, which is corresponding to a condensate of diethyl 2,3-dihydro-2-(hydroxymethyl)thieno[3,4-b][1,4]dioxine-5,7-dicarboxylate **IV** or 3-hydroxy-3,4-dihydro-2H-thieno[3,4-b][1,4]dioxepine-6,8-dicarboxylic acid diethyl ester **V** with ebibromhydrine.

### d) Synthesis of a mixture of 2-(hydroxymethyl)-2,3-dihydrothieno[3,4-b]-1,4-dioxine-5,7-dicarboxylic acid VI and 3-hydroxy-3,4-dihydro-2H-thieno[3,4-b][1,4]dioxepine-6,8-dicarboxylic acid VII

A solution of 218,8 g potassium hydroxide (5,5 mol) in 2 000 ml water was prepared and 200 g of a mixture of 77 % of diethyl 2,3-dihydro-2-(hydroxymethyl)thieno[3,4-b][1,4]dioxine-5,7-dicarboxylate **IV** and 23 % of 3-hydroxy-3,4-dihydro-2H-thieno[3,4-b][1,4]dioxepine-6,8-dicarboxylic acid diethyl ester **V** (0,89 mol) were continuously added. The mixture was heated at reflux for 2 hours. Ethanol and water (250 ml) were then distilled off. The reaction mixture was then cooled to 15 °C when 465 ml of hydrochloric acid were added. The mixture was then cooled to 0 °C and mixed for 2 hours. The precipitate was filtered off and dried under reduced pressure. 125 g of a mixture 2-(hydroxymethyl)-2,3-dihydrothieno[3,4-b]-1,4-dioxine-5,7-dicarboxylic acid **VI** and 3-hydroxy-3,4-dihydro-2H-thieno[3,4-b][1,4]dioxepine-6,8-dicarboxylic acid **VII** was obtained, corresponding to a yield of 54 %. According to the HPLC analysis, the molar ratio of 2-(hydroxymethyl)-2,3-dihydrothieno[3,4-b]-1,4-dioxine-5,7-dicarboxylic acid **VI** and 3-hydroxy-3,4-dihydro-2H-thieno[3,4-b][1,4]dioxepine-6,8-dicarboxylic acid **VII** was 75 to 25 %.

### e) Synthesis of a mixture of (2,3-dihydrothieno[3,4-b][1,4]dioxin-2-yl)methanol VIII and 3,4-dihydro-2H-thieno[3,4-b][1,4]dioxepin-3-ol IX

A mixture of 690 ml of triethanolamine, 115 g of a mixture of 2-(hydroxymethyl)-2,3-dihydrothieno[3,4-b]-1,4-dioxine-5,7-dicarboxylic acid **VI** and 3-hydroxy-3,4-dihydro-2H-thieno[3,4-b][1,4]dioxepine-6,8-dicarboxylic acid **VII** (0,44 mol) and 12,4 g of CuO.Cr₂O₃.BaO was heated to 180 °C and stirred for 3 hours at this temperature. The resulting slurry was then cooled to 150 °C and poured into 1 700 ml of water. The catalyst was filtered off and the filtrate extracted several times by means of 70 ml of trichloromethane. The collected trichloromethane fractions were dried by sodium sulphate. Thereafter, trichloromethane was removed under reduced pressure. 65 g of a mixture (2,3-dihydrothieno[3,4-b][1,4]dioxin-2-yl)methanol **VIII** and 3,4-dihydro-2H-thieno[3,4-b][1,4]dioxepin-3-ol **IX** was obtained, corresponding to a yield of 86 %. According to the HPLC analysis, the molar ratio of (2,3-dihydrothieno[3,4-b][1,4]dioxin-2-yl)methanol **VIII** and 3,4-dihydro-2H-thieno[3,4-b][1,4]dioxepin-3-ol **IX** was 87,5 to 12,5 %.

### Example 1

### Influence of purity of mixture of diethyl 2,3-dihydro-2-(hydroxymethyl)thieno[3,4-b][1,4]dioxine-5,7-dicarboxylate IV and 3-hydroxy-3,4-dihydro-2H-thieno[3,4-b][1,4]dioxepine-6,8-dicarboxylic acid diethyl ester V on molar ratio of (2,3-dihydrothieno[3,4-b][1,4]dioxin-2-yl)methanol VIII and 3,4-dihydro-2H-thieno[3,4-b][1,4]dioxepin-3-ol IX

Mixture of diethyl 2,3-dihydro-2-(hydroxymethyl)thieno[3,4-b][1,4]dioxine-5,7-dicarboxylate **IV** and 3-hydroxy-3,4-dihydro-2H-thieno[3,4-b][1,4]dioxepine-6,8-dicarboxylic acid diethyl ester **V** was purified by extraction with cyclohexane. This extraction step removed impurities with formulas **X** and **XI,** i.e., condensates of diethyl 2,3-dihydro-2-(hydroxymethyl)thieno[3,4-b][1,4]dioxine-5,7-dicarboxylate **IV** or 3-hydroxy-3,4-dihydro-2H-thieno[3,4-b][1,4]dioxepine-6,8-dicarboxylic acid diethyl ester **V** with ebibromhydrine. The amount of these impurities was determined by GC/MS analysis. Samples with different amounts of said impurities were obtained and used for the synthetic procedure described in the comparative example. The molar ratios of (2,3-dihydrothieno[3,4-b][1,4]dioxin-2-yl)methanol **VIII** and 3,4-dihydro-2H-thieno[3,4-b][1,4]dioxepin-3-ol **IX** (EDOT MeOH : ProDOT OH) in the final products were determined by HPLC. The results are shown in the next table.

| Amount of impurities of formulas X and XI in mixture of IV and V [sq. % by GC/MS] | Molar Ratio EDOT MeOH : ProDOT OH by HPLC |
|---|---|
| 10,0 | 87,5:12,5 |
| 9,0 | 88,5:11,5 |
| 7,7 | 89,4:10,6 |
| 2,5 | 93,9:6,1 |
| 1,2 | 98,9:1,1 |
| 0,0 | 100,0:0,0 |

### Example 2

### Procedure for the synthesis of mixture of IV and V free from impurity with M = 372 and influence on the molar ratio of (2,3-dihydrothieno[3,4-b][1,4]dioxin-2-yl)methanol VIII and 3,4-dihydro-2H-thieno[3,4-b][1,4]dioxepin-3-ol IX

A solution of 200 g of diethyl 3,4-dihydroxy-thiophene-2,5-dicarboxylic acid diethyl ester **III** (0,768 mol), prepared according to the procedure described in the comparative example in 1 250 ml of ethanol was prepared by mixing and heating at reflux. A mixture of 32,8 g potassium carbonate (0,154 mol) in 1 000 ml water was slowly added to 231 g of epibromohydrin (1,69 mol) within 3 hours. The mixture was then left under reflux for 2 hours, then cooled at 30 °C and poured into a mixture of 2 200 ml water and 600 ml of hydrochloric acid. The resulting suspension was cooled at 0 °C and stirred for 3 hours at this temperature. The precipitate was filtered off, washed with water and dried at 65 °C. 211 g of a mixture of diethyl 2,3-dihydro-2-(hydroxymethyl)thieno[3,4-b][1,4]dioxine-5,7-dicarboxylate **IV** and 3-hydroxy-3,4-dihydro-2H-thieno[3,4-b][1,4]dioxepine-6,8-dicarboxylic acid diethyl ester **V** was obtained, corresponding to a yield of 87 %. HPLC analysis proved that the product consisted of 78 % of diethyl 2,3-dihydro-2-(hydroxymethyl)thieno[3,4-b][1,4]dioxine-5,7-dicarboxylate **IV** and 22 % of 3-hydroxy-3,4-dihydro-2H-thieno[3,4-b][1,4]dioxepine-6,8-dicarboxylic acid diethyl ester **V**. By GC/MS no impurities corresponding to formulas **X** and **XI** were detected. This product was further processed according to the procedure described in the comparative example to obtain 86,0 g of pure (2,3-dihydrothieno[3,4-b][1,4]dioxin-2-yl)methanol **VIII.** Neither HPLC, nor GC/MS revealed any indication of present 3,4-dihydro-2H-thieno[3,4-b][1,4]dioxepin-3-ol **IX**. The yield calculated based on the amount of the starting material diethyl 3,4-dihydroxythiophene-2,5-dicarboxylate **III** was 65 %.

### Example 3

### Synthesis of (2,3-dihydrothieno[3,4-b][1,4]dioxin-2-yl)methanol VIII in quinoline medium

A mixture of diethyl 2,3-dihydro-2-(hydroxymethyl)thieno[3,4-b][1,4]dioxine-5,7-dicarboxylate **IV** and 3-hydroxy-3,4-dihydro-2H-thieno[3,4-b][1,4]dioxepine-6,8-dicarboxylic acid diethyl ester **V** prepared according to Example 2 was used for the synthesis of a mixture of 2-(hydroxymethyl)-2,3-dihydrothieno[3,4-b]-1,4-dioxine-5,7-dicarboxylic acid **VI** and 3-hydroxy-3,4-dihydro-2H-thieno[3,4-b][1,4]dioxepine-6,8-dicarboxylic acid **VII** described in the comparative example. 150 g of mixture **VI** and **VII** were obtained containing 78 % of 2-(hydroxymethyl)-2,3-dihydrothieno[3,4-b]-1,4-dioxine-5,7-dicarboxylic acid **VI** and 22 % of 3-hydroxy-3,4-dihydro-2H-thieno[3,4-b][1,4]dioxepine-6,8-dicarboxylic acid **VII**. This intermediate was used for the last step. A mixture of 750 ml quinoline, 115 g of a mixture of 2-(hydroxymethyl)-2,3-dihydrothieno[3,4-b]-1,4-dioxine-5,7-dicarboxylic acid **VI** and 3-hydroxy-3,4-dihydro-2H-thieno[3,4-b][1,4]dioxepine-6,8-dicarboxylic acid **VII** (0,44 mol), and 12,4 g of CuO.Cr₂O₃.BaO was heated at 180 °C and stirred for 3 hours at this temperature. The mixture was then cooled at 150 °C and poured into 1 700 ml of water. The catalyst was filtered off and filtrate extracted several times by 70 ml of toluene. The collected toluene fractions were dried by sodium sulphate, after which the toluene was removed under reduced pressure. 60 g of a mixture (2,3-dihydrothieno[3,4-b][1,4]dioxin-2-yl)methanol **VIII** and 3,4-dihydro-2H-thieno[3,4-b][1,4]dioxepin-3-ol **IX** were obtained, corresponding to a yield of 79 %. According to HPLC the molar ratio of (2,3-dihydrothieno[3,4-b][1,4]dioxin-2-yl)methanol **VIII** and 3,4-dihydro-2H-thieno[3,4-b][1,4]dioxepin-3-ol **IX** was 80,5 to 19,5 %.

### Example 4

### Purification of the hexyl ether of (2,3-dihydrothieno[3,4-b][1,4]dioxin-2-yl)methanol VIII by column chromatography

Raw hexyl ether of (2,3-dihydrothieno[3,4-b][1,4]dioxin-2-yl)methanol **VIII** prepared according to the procedure described in WO 2008/064878 from 6-bromohexane and (2,3-dihydrothieno[3,4-b][1,4]dioxin-2-yl)methanol **VIII** contained a mixture of the desired monomer, the starting compound (2,3-dihydrothieno[3,4-b][1,4]dioxin-2-yl)methanol **VIII,** 3,4-dihydro-2H-thieno[3,4-b][1,4]dioxepin-3-ol **IX**, 6-bromohexane, aliphatic compounds originating from 6-bromohexane decomposition, oligomeric impurities originating from (2,3-dihydrothieno[3,4-b][1,4]dioxin-2-yl)methanol **VIII** and/or 3,4-dihydro-2H-thieno[3,4-b][1,4]dioxepin-3-ol **IX** and/or the desired monomer. This mixture was purified by column chromatography under low pressure. 45 g of the raw mixture prepared from 34,4 g (0,20 mol) of a mixture of (2,3-dihydrothieno[3,4-b][1,4]dioxin-2-yl)methanol **VIII** and 3,4-dihydro-2H-thieno[3,4-b][1,4]dioxepin-3-ol **IX** in a molar ratio of 87,5:12,5 % was put on top of a column (diameter 4 cm, length 100 cm) containing 150 g silica (Merck 9385, 230 - 400 Mesh, 60Å) with the mobile phase being hexane:dichlormethane 2:1. Under a pressure of 0,5 MPa fractions of 100 ml were collected and the presence of the desired monomer was checked by TLC analysis. The first 10 fractions contained the desired monomer, the second 10 fractions contained the starting compound (2,3-dihydrothieno[3,4-b][1,4]dioxin-2-yl)methanol **VIII.** 25,65 g (yield 50,0 %) and 1,55 g (yield 4,5 %) of evaporation residue were obtained from the first and second fractions, respectively. The first fraction was identified by GC/MS as a mixture of 81,5 % of hexyl ether of (2,3-dihydrothieno[3,4-b][1,4]dioxin-2-yl)methanol **VIII,** 13,5 % of hexyl ether of 3,4-dihydro-2H-thieno[3,4-b][1,4]dioxepin-3-ol **IX,** 0,8 % of 6-bromhexane, 0,5 % of di-n-hexylether, 3,6 % of di-n-hexylsulfide and 0,2 % of di-n-hexyldisulfide. The second fraction was identified by HPLC as a mixture of (2,3-dihydrothieno[3,4-b][1,4]dioxin-2-yl)methanol **VIII** (85 %) and hexyl ether of (2,3-dihydrothieno[3,4-b][1,4]dioxin-2-yl)methanol **VIII** (15 %). This mixture could be further processed for the next synthetic batch.

### Example 5

### Electrochromic layer prepared from a monomer VIII purified by column chromatography

12.71 g of a monomer mixture containing 93 % of hexyl ether of (2,3-dihydrothieno[3,4-b][1,4]dioxin-2-yl)methanol **VIII** and 5 % of hexyl ether of 3,4-dihydro-2H-thieno[3,4-b][1,4]dioxepin-3-ol **IX** are mixed with 18.53 g of n-butanol, 2.22 g of n-propylamine, 0.99 g of 3-aminopropyl triethoxysilane, and 124.58 g of a solution of water-free iron(III) tosylate inn-butanol (Clevios C-B 40R from Heraeus). The mixture is diluted with another 81.00 g 10 of n-butanol and filled in a glass bottle with screw-cap and a suitable grommet. Prior to mixing, all components are pre-cooled to -10°C in a thermostat. Immediately after mixing, the mixture is vigorously stirred for one minute and then held at -10° C, to obtain a ready-to-apply colloidal solution with a potlife of at least one day.

A conducting PET-ITO film having a thickness of 125 µm, a width of 305 mm, a length of 150 meters, and a sheet resistance of 60 Ohm/sq on 6" paper core (by CP Films/Solutia, Inc.) is inserted in a Click&Coat® modular line provided by Coatema Coating Machinery GmbH. The liquid working electrode composition is transferred to a slot die by means of a micro annular gear pump and continuously applied to the substrate surface, resulting in a wet film. The wet film is allowed to level and pre-polymerize for 5 minutes before it is transferred into an oven where polymerization is completed at 120 °C within 3 min. The polymer film is then allowed to relax for another 3 min and cooled down to room temperature. A longer drying time can be useful to improve film adhesion. The humidity level is adjusted between 25 and 35 %rH in both the slot die application and pre-polymerization sections of the coating line. The film can be re-wound with low tension using a protective polyethylene film and stored under ambient conditions.

A total amount of 240 ml coating solution is sufficient to equip 40 metres of the conducting substrate (305 mm in width) used in the present example with an electrochromic conducting polymer coating having a calculated wet film thickness of 9.95 µm. The electrochromic conducting polymer coating can be applied to other plastic films (e.g. plain PET) according to the same method.

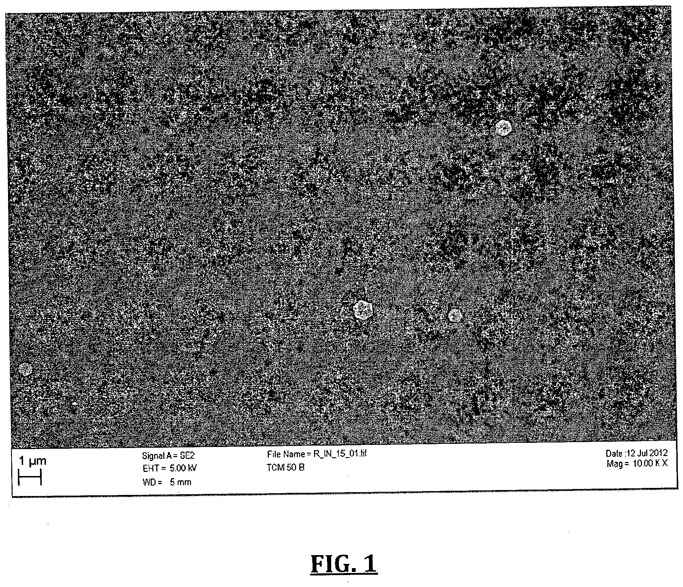

The scanning electron micrograph in Fig. 1 shows the morphology of such a coating. The apparent circular structures have diameters in the 500-1000 nm range, which may result in light scattering (hazy films) and poor adhesion when wetted with (or immersed in) liquid media such as rinsing liquids or liquid electrolytes.

### Industrial applicability

The monomer based on thiophene of formula (2,3-dihydrothieno[3,4-b][1,4]dioxin-2-yl)methanol can be used for preparation of thin conductive layers, e.g. active layers of electrochromic systems.

## Claims

1. A method for the preparation of a monomer based on thiophene of formula (2,3-dihydrothieno[3,4-b][1,4]dioxin-2-yl)methanol (VIII), where the monomer is synthetized according to the following reaction scheme 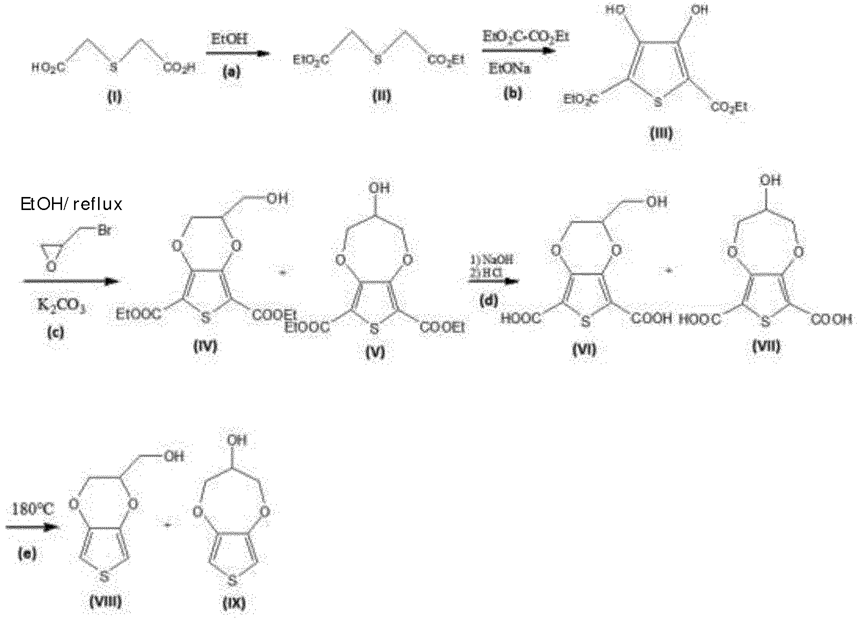 where in the step (c) of the reaction a mixture of 3,4-dihydroxy-thiophene-2,5-dicarboxylic acid diethyl ester (III), epibromhydrin and potassium carbonate is used to prepare a mixture of intermediates: diethyl 2,3-dihydro-2-(hydroxymethyl)thieno[3,4-b][1,4]dioxine-5,7-dicarboxylate (IV) and 3-hydroxy-3,4-dihydro-2H-thieno[3,4-b][1,4]dioxepine-6,8-dicarboxylic acid diethyl ester (V), **characterized in that** in step (c) of the reaction epibromhydrin and potassium carbonate are dosed in intervals of 120 to 240 minutes into the solution of 3,4-dihydroxy-thiophene-2,5-dicarboxylic acid diethyl ester (III), while the molar ratio of 3,4-dihydroxy-thiophene-2,5-dicarboxylic acid diethyl ester (III) and epibromhydrin is in the range 1:20 to 1:25, and after the dosing is completed the mixture is stirred for the maximum period of 180 minutes, where the mixture of such intermediates contains less than 0.1 % wt. of impurities of general formula (X, XI) 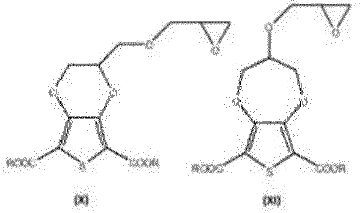 created as a product of condensation of a mixture of the compounds (IV and V) with epibromhydrin and the mixture of the compounds (IV and V) is subsequently used in the reaction steps (d) and (e) to prepare the resulting monomer (VIII) and the byproduct 3,4-dihydro-2H-thieno[3,4-b][1,4]dioxepin-3-ol (IX), where the resulting monomer (VIII) contains less than 1 % wt. of the mentioned byproduct (IX).

2. A method according to claim 1 **characterized in that,** in order to further reduce the content of impurities (X, XI), the mixture of the compounds (IV and V) is washed with one or mixture of the non-polar solvent from the group cyclohexane, toluene, benzene or mixtures thereof.

## Patentansprüche

1. Vorgehensweise der Monomer-Zubereitung auf Thiophen-Basis mit der chemischen Formel (2,3-Dihydrothieno[3,4-][1,4]dioxin-2-yl)methanol (VIII), wo das Monomer nach dem folgendem Reaktionsschema synthetisiert wird: 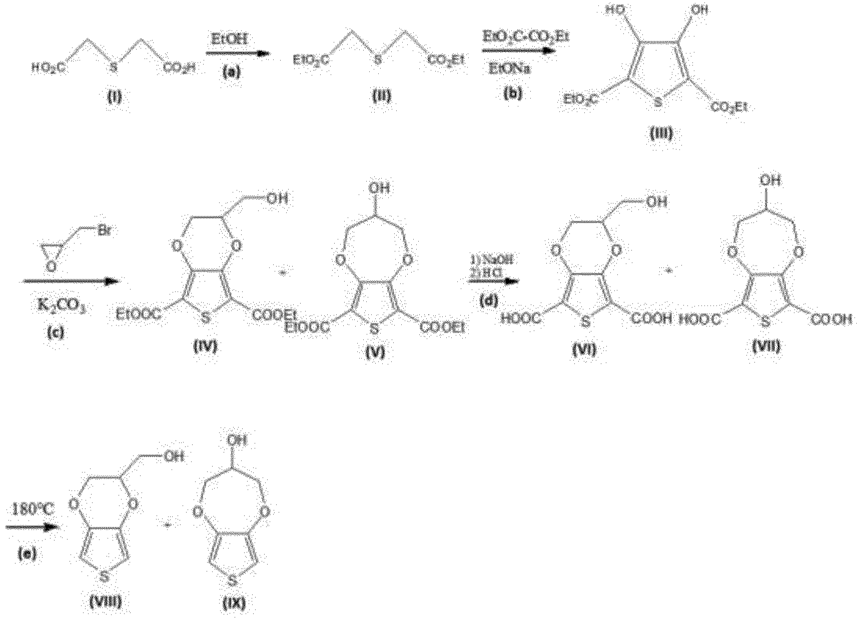 wo beim Schritt (c) die Reaktion des Gemisches aus Diethylester der 3,4-dihydroxy-thiofen-2,5-Dicarboxylsäure (III), Epibromhydrin und Kaliumcarbonat für die Zubereitung nachfolgender Zwischenprodukt-Gemisches genutzt wird: Diethyl 2,3-dihydro-2-(hydroxymethyl)thieno[3,4-b][1,4]dioxin-5,7-Dicarboxylat (IV) und Diethylester der 3-hydroxy-3,4-dihydro-2H-thieno[3,4-b][1,4]dioxepin-6,8-Dikarboxylsäure (V), **dadurch geennzeichnet, dass** beim Schritt (c) die Reaktion von Epibromhydrin und Kaliumcarbonat in Intervallen von 120 bis 240 Minuten in die Lösung von Diethylester der 3,4-dihydroxy-thiofen-2,5-Dikarboxylsäure (III) dosiert wird, wobei das Molarverhältnis von Diethylesters der 3,4-dihydroxy-thiofen-2,5-Dikarboxylsäure (III) und Epibromhydrin im Bereich von 1:20 bis 1:25 ist, und nach der Beendigung der Dosierung das Gemisch in Dauer von maximal 180 Minuten gemischt wird, wobei das Zwischenprodukt-Gemisch weniger als 0,1 Gew.- % Verunreinigung mit der allgemeinen Formel (X, XI) enthält, 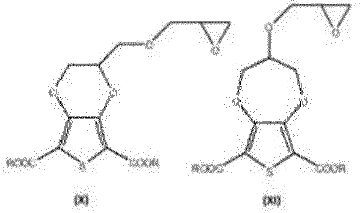 die als Produkt der Kondensation des Gemisches von Verbindungen (IV und V) mit Epibromhydrin entstanden sind, und das Gemisch der Verbindungen (IV und V) weiterhin bei den Reaktionsschritten (d) und (e) für die Zubereitung des Endmonomers (VIII) und des Nebenprodukts 3,4-dihydro-2H-thieno[3,4-b][1,4]dioxepin-3-ol (IX) verwendet wird, wobei das Endmonomer (VIII) weniger als 1 Gew.-% des angeführten Nebenprodukts (IX) enthält.

2. Vorgehensweise nach Anspruch 1, **dadurch gekennzeichnet, dass** um den Inhalt der Verunreinigungen (X, XI) weiterhin reduzieren zu können, wird das Gemisch der Verbindungen (IV und V) mit einem oder einem Gemisch von nicht polaren, Cycloxexan, Toluen, Benzol oder deren Gemische enthaltenden Lösungsmitteln oder einem Gemisch solcher Lösungen gespült.

## Revendications

1. La méthode pour la préparation d'un monomère à base de thiophène réponant à la formule (2,3-dihydrothiéno [3,4-b] [1,4]dioxine-2-yl)méthanol (VIII) où le monomère est synthétisé selon le schéma réactionnel suivant 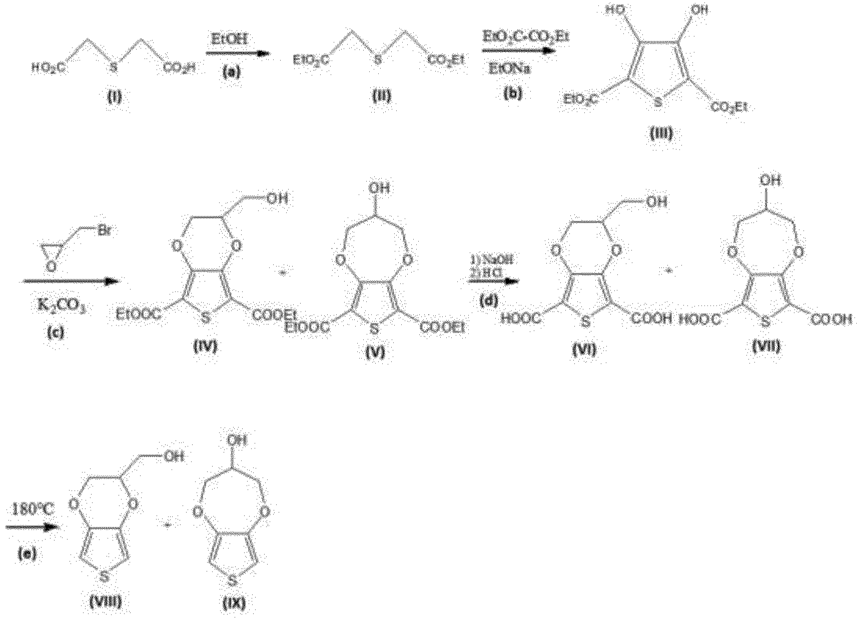 où à l'étape (c) de la réaction, un mélange d'ester diéthylique d'acide 3,4-dihydroxy-thiophène-2,5-dicarboxylique (III), l'épibromhydrine et le carbonate de potassium sontt utilisés pour préparer un mélange d'intermédiaires : diéthyl 2, 3-dihydro-2-(hydroxyméthyl)thieno[3,4-b][1,4]dioxine-5,7-dicarboxylate (IV) et d'ester diéthylique de l'acide 3-hydroxy-3,4-dihydro-2H-thiéno [3,4-b] [1,4] dioxépine-6,8-dicarboxylique (V), **se caractérisant dans celle-ci,** à l'étape (c) de la réaction l'épibromhydrine et le carbonate de potassium sont dosés par intervalles de 120 à 240 minutes dans la solution d'ester diéthylique acide 3,4-dihydroxy-thiophène-2, 5-dicarboxylique (III), tandis que le ratio molaire d'ester diéthylique d'acide (III) 3,4-dihydroxy-thiophène-2,5-dicarboxylique (III) et l'épibromhydrine est dans la gamme 1:20 à 1:25, et une fois le dosage terminé, le mélange est agité pendant la période maximale de 180 minutes, où le mélange de ces intermédiaires contient moins de 0,1 % en poids d'impuretés de formule générale (X, XI) 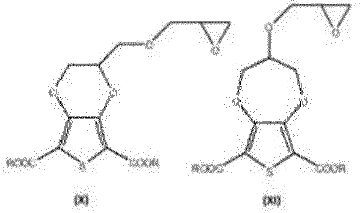 créé en tant que produit de condensation d'un mélange de composés (IV et V) avec l'épibromhydrine et le mélange de composés (IV et V) est ensuite utilisé dans les étapes réactionnelles (d) et (e) pour préparer le monomère résultant (VIII) et le sous-produit 3,4-dihydro-2H-thiéno [3,4-b] [1,4] dioxépin-3-ol (IX), où le monomère résultant (VIII) contient moins de 1% en poids du sous-produit mentionné (IX).

2. La méthode selon la revendication 1, **se caractérisant dans celle-ci,** afin de réduire la teneur en impuretés (X, XI), le mélange de composés (IV et V) est lavé avec un solvant non polaire ou son mélange du groupe de cyclohexane, toluène, benzène ou leurs mélanges.
